# EUROPEAN PATENT APPLICATION

(11) **EP 4 368 602 A1**
(43) Date of publication of application: **15.05.2024**
(21) Application number: 22837354.4
(22) Date of filing: 26.05.2022
(51) Int. Cl.: C07B 35/02, C07C 45/41, C07C 213/02, C07C 215/76, C07C 49/403, C07B 61/00, B01J 19/24, B01J 23/44, B01J 27/053

(54) **GAS-LIQUID REACTION METHOD**

(30) Priority: 06.07.2021 JP 2021112315
(71) Applicant: CATALER CORPORATION, Kakegawa-shi, Shizuoka 437-1492 (JP)
(72) Inventor: SAITO, Yusuke, Kakegawa-shi, Shizuoka 437-1492 (JP); MIZUKAMI, Tomohito, Kakegawa-shi, Shizuoka 437-1492 (JP); MAKI, Yohei, Kakegawa-shi, Shizuoka 437-1492 (JP)
(74) Representative: Wallinger Ricker Schlotter Tostmann
(86) International application number: PCT/JP2022/021648
(87) International publication number: WO 2023/281933

(57) **Abstract**

Provided is a reaction method that includes, in a gas-liquid reaction performed using a solid catalyst, alternately allowing a gaseous raw material 3 that includes a first raw material and a liquid raw material 5 that includes a second raw material to pass through a reaction field 1 that holds the solid catalyst, and reacting the first and second raw materials on the solid catalyst.

## Description

### FIELD

The present invention relates to a gas-liquid reaction method.

### BACKGROUND

Gas-liquid reactions using solid catalysts are conducted by placing a solid catalyst in a liquid phase containing a liquid phase reactant and supplying a gas phase reactant to it. Since the liquid phase reactant is in constant contact with the solid catalyst, the gas phase reactant reaches the solid catalyst so that gas-liquid reaction is promoted.

If the solubility of the gas phase reactant in the liquid phase is low, however, it becomes difficult to supply the gas phase reactant to the solid catalyst, thus lowering efficiency of the gas-liquid reaction.

Strategies for increasing gas-liquid reaction efficiency in the prior art have included raising the pressure of the gas phase to increase the liquid solubility of gas phase reactants, and using a trickle-bed reactor to improve contact efficiency between liquid phase reactants, gas phase reactants and solid catalyst.

PTL 1, for example, discloses a trickle-bed reactor provided with a heat transfer plate having a predetermined area.

### [CITATION LIST]

### [PATENT LITERATURE]

[PTL 1] Japanese Unexamined Patent Publication No. 2012-005966

### SUMMARY

### [TECHNICAL PROBLEM]

It is an object of the invention to provide a novel method that allows a gas-liquid reaction using a solid catalyst to be carried out at high efficiency.

### [SOLUTION TO PROBLEM]

The present invention is as follows.

### <Aspect 1>

A reaction method for a gas-liquid reaction using a solid catalyst, wherein the method includes:
alternately flowing a gaseous starting material containing a first starting material and a liquid starting material containing a second starting material through a reaction field holding a solid catalyst, to react the first starting material and second starting material on the solid catalyst.

### <Aspect 2>

The reaction method according to aspect 1, wherein each feed rate for the gaseous starting material, in terms of the volume of the gaseous starting material at the reaction temperature, is either equal to the volume of the reaction field or greater than the volume of the reaction field.

### <Aspect 3>

The reaction method according to aspect 1 or 2, wherein each feed rate for the liquid starting material, in terms of the volume of the liquid starting material, is either equal to the volume of the reaction field or greater than the volume of the reaction field.

### <Aspect 4>

The reaction method according to any one of aspects 1 to 3, wherein:
the reaction field is a honeycomb substrate, and
the solid catalyst is held on the honeycomb substrate.

### <Aspect 5>

The reaction method according to any one of aspects 1 to 4, wherein the liquid starting material that has been flowed through the reaction field is recovered and reflowed.

### <Aspect 6>

The reaction method according to any one of aspects 1 to 5, wherein:
the first starting material is hydrogen, and
the second starting material is a hydrogen-reducible compound.

### <Aspect 7>

The reaction method according to any one of aspects 1 to 5, wherein:
the first starting material is oxygen or air, and
the second starting material is an oxygen-oxidizable or air-oxidizable compound.

### <Aspect 8>

A reaction system that carries out the reaction method according to any one of aspects 1 to 7, wherein the reaction system has:
a reaction field,
a gaseous starting material housing unit for housing of the gaseous starting material,
a liquid starting material housing unit for housing of the liquid starting material,
a gaseous starting material feeder for flow of the gaseous starting material through the reaction field in an intermittent manner, and
a liquid starting material feeder for flow of the liquid starting material through the reaction field in an intermittent manner.

### <Aspect 9>

The reaction system according to aspect 8, which has a construction in which the liquid starting material that has been flowed through the reaction field is recovered and reflowed.

### [ADVANTAGEOUS EFFECTS OF INVENTION]

The reaction method of the invention allows gas-liquid reaction using a solid catalyst to be carried out at high efficiency.

### BRIEF DESCRIPTION OF DRAWINGS

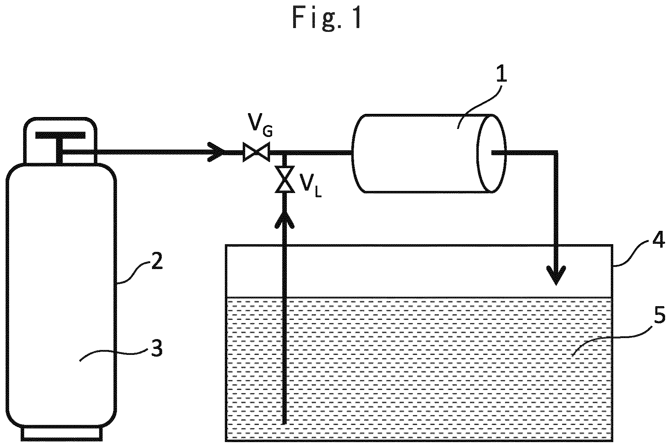

Fig. 1 is a schematic diagram showing the reaction system used in the Examples and Comparative Examples, in overview.

### DESCRIPTION OF EMBODIMENTS

### <Reaction method>

The reaction method of the invention is a reaction method for a gas-liquid reaction using a solid catalyst, wherein the method includes:
alternately flowing a gaseous starting material containing a first starting material and a liquid starting material containing a second starting material through a reaction field holding a solid catalyst, to react the first starting material and second starting material on the solid catalyst.

### <Reaction using reaction method of the invention>

The reaction carried out using the reaction method of the invention is a gas-liquid reaction between the first starting material and second starting material on a solid catalyst.

### (First starting material)

The first starting material is a gaseous reactant, and in the method of the invention, it is supplied to the reaction field in a gaseous state. That the first starting material is a "gaseous reactant" means only that it is a gas at the temperature and pressure at the reaction field when supplied to the reaction field, and it does not need to be gaseous at ordinary temperature and pressure.

The first starting material may be supplied to the method of the invention directly as a gas, or it may be supplied to the method of the invention after having been diluted with an appropriate dilution gas.

The dilution gas may be any gas that does not react with the first starting material, second starting material and solid catalyst.

The concentration of the first starting material in the gas may be set as appropriate depending on the type of gas-liquid reaction, and the type and scale of the reaction field.

The first starting material may be a single type or a mixture of two or more types.

### (Second starting material)

The second starting material is included in the liquid starting material, and preferably dissolves in the liquid starting material. The second starting material itself may be a solid, liquid or gas, and it may also be in a supercritical state. When the second starting material itself is in a state other than liquid, it may be supplied to the method of the invention after having been dissolved in an appropriate solvent. When the second starting material itself is a liquid, it may be either supplied to the method of the invention directly or supplied to the method of the invention after having been dissolved in an appropriate solvent.

The solvent of the liquid starting material may be a liquid that can dissolve or disperse the second starting material, and that does not react with the first starting material, second starting material and solid catalyst.

The concentration of the second starting material in the liquid starting material may be set as appropriate depending on the type of gas-liquid reaction, and the type and scale of the reaction field.

The second starting material may be a single type or a mixture of two or more types.

### (Gas-liquid reaction)

There is no restriction on the type of gas-liquid reaction between the first starting material and second starting material. For example, it may be a reduction reaction, addition reaction, oxidation reaction, substitution reaction, polymerization reaction, condensation polymerization reaction, condensation reaction, homocoupling reaction, cross-coupling reaction, dehydrating reaction, elimination reaction, decomposition reaction, electrocyclic reaction, cyclization reaction, electron transfer reaction, transfer reaction or halogenation reaction.

The following are examples of gas-liquid reactions between the first starting material and second starting material.

Cases where:
the first starting material is hydrogen, the second starting material is a hydrogen-reducible compound, and the gas-liquid reaction is hydrogen reduction reaction;
the first starting material is hydrogen, the second starting material is a compound with a carbon-carbon unsaturated bond, and the gas-liquid reaction is hydrogenation reaction;
the first starting material is carbon monoxide and hydrogen, the second starting material is an alkene, and the gas-liquid reaction is hydroformylation reaction;
the first starting material is an aryl halide or haloalkane, the second starting material is a terminal alkyne and the gas-liquid reaction is Sonogashira cross-coupling reaction;
the first starting material is a terminal alkyne, the second starting material is an aryl halide or haloalkane, and the gas-liquid reaction is Sonogashira cross-coupling reaction;
the first starting material is air or oxygen, the second starting material is an oxygen-oxidizable or air-oxidizable compound, and the gas-liquid reaction is air oxidation or oxygen oxidation reaction; and
the first starting material is an alcohol, the second starting material is a carboxylic acid, and the gas-liquid reaction is esterification reaction.

The compound with a hydrocarbon group in the second starting material for the aldehyde derivative production reaction may be a compound with an alkyl group, and in particular it may be an aromatic compound with an alkyl group. The compound with an OH group in the second starting material for the carboxylic acid derivative production reaction may be a compound with an OH group bonded to a primary carbon atom, and in particular it may be an aromatic compound with an OH group bonded to a primary carbon atom.

A specific example for the hydrogen reduction reaction is reduction reaction of a nitro group to an amino group.

Specific examples of oxygen oxidation reaction or air oxidation reaction are:
air oxidation or oxygen oxidation reaction of a compound with a hydrocarbon group to an aldehyde derivative;
air oxidation or oxygen oxidation reaction of a primary alcohol to an aldehyde; and
air oxidation or oxygen oxidation reaction of a primary alcohol to a carboxylic acid.

An example of a hydrogenation reaction is hydrogenation reaction on an aromatic ring.

### (Solid catalyst)

The solid catalyst is a solid with the ability to promote gas-liquid reaction between the first starting material and second starting material. The term "solid catalyst" as used herein includes the concept of a solid having the function of lowering the activation energy in the gas-liquid reaction between the first starting material and second starting material to directly promote gas-liquid reaction, as well as a solid having the function of adsorption/desorption of the first starting material or second starting material, or both, to increase the contact frequency between the first starting material and second starting material, thereby indirectly promoting the gas-liquid reaction.

The form of the solid catalyst is not restricted so long as it is solid and has a function of promoting gas-liquid reaction between the first starting material and second starting material. For example, it may be a catalyst composed of a single chemical species, a catalyst composition composed of multiple chemical species or a supported catalyst having a catalytically active species supported on a support, or the catalytically active species itself may form a structure (such as a sheet or wire). The solid catalyst may also include a component that adsorbs gases (especially the first starting material).

The type of solid catalyst may be selected as appropriate for the gas-liquid reaction to be carried out using the reaction method of the invention. Specifically, the solid catalyst may be a metal, metal oxide, metal hydroxide or metal halide, or a solid acid or solid base other than these. The metal may be a precious metal or a precious metal alloy, and in particular it may be a platinum-group metal such as palladium, platinum or rhodium, or an alloy of any one or more of these. These metals may be held at the reaction field in a state supported on an appropriate support (such as metal oxide particles).

### <Reaction field>

Throughout the present specification, the term "reaction field" means a space where the first reactant and second reactant and solid catalyst contact to produce gas-liquid reaction. The reaction field may be a honeycomb substrate, reactor, column, wire, mesh or particle surfaces, for example.

The solid catalyst is held in the reaction field.

The reaction field may be:
a fixed bed reaction field with the solid catalyst held in a fixed manner;
a fluidized bed reaction field with the solid catalyst held in a fluidized manner; or
a circulating fluidized bed reaction field wherein the solid catalyst is circulated in and out of the reaction field and supplied for gas-liquid reaction at the reaction field, after which it is exported out of the reaction field, regenerated outside the reaction field, and returned back to the reaction field.

More specifically, the reaction field for the reaction method of the invention may be a honeycomb substrate, with the solid catalyst held on the honeycomb substrate. The solid catalyst held on the honeycomb substrate may be held by being directly fixed on the cell walls of the honeycomb substrate, or it may be held by being fixed in a coating layer formed on the cell walls of the honeycomb substrate.

The honeycomb substrate preferably has multiple cell flow channels partitioned by porous cell walls. The honeycomb substrate may be a straight flow type or a wall flow type of honeycomb substrate. With a straight flow type of honeycomb substrate, multiple cell flow channels are connected in the lengthwise direction of the substrate from the upstream end to the downstream end of the exhaust gas flow. A wall flow type honeycomb substrate has a construction with an inlet side cell flow channel having the upstream end of the exhaust gas flow open and the downstream end closed, and an outlet side cell flow channel having the upstream end of the exhaust gas flow closed and the downstream end open, whereby exhaust gas flowing into the inlet side cell flow channel passes through the porous cell walls and is discharged from the outlet side cell flow channel.

The structural material of the honeycomb substrate may be ceramic, metal or synthetic resin, or a combination of the same. A ceramic may be cordierite, and a metal may be stainless steel. A metal honeycomb substrate may be integrally formed, or constructed with a perforated or non-perforated corrugated metal sheet and a perforated or non-perforated flat metal sheet layered and wound together, or it may be a wound body of an expanded metal.

The reaction field for the reaction method of the invention is preferably a straight flow type or wall flow type honeycomb substrate wherein the cell flow channels are partitioned by ceramic (such as cordierite) porous cell walls. When a honeycomb substrate is used as the reaction field, it is possible to easily increase the specific surface area of the solid catalyst while also increasing the probability of contact between the first starting material and second starting material supplied to the reaction field, and the solid catalyst, thereby increasing the efficiency of the gas-liquid reaction.

When the solid catalyst is to be held by being directly fixed on the cell walls of a honeycomb substrate, it can be produced by contacting the cell walls of the honeycomb substrate with a coating solution containing the solid catalyst or its precursor, and firing if necessary.

When the solid catalyst is to be held by being fixed in a coating layer formed on the cell walls of a honeycomb substrate, it can be produced by contacting the cell walls of the honeycomb substrate with a coating solution containing the main material of the coating layer (such as metal oxide particles), and the solid catalyst or its precursor, and firing if necessary. In this case, the coating layer may be a single layer or may have a multilayer structure with two or more layers, and it may also have multiple regions with different coating layer structures from one end side to the other side in the lengthwise direction of the honeycomb substrate.

One, two or more reaction fields may be used in the reaction method of the invention. When two or more reaction fields are used, the form of each reaction field and the manner in which the solid catalyst is held at each reaction field, may be the same or different. Two or more reaction fields may also be linked in series or parallel. In the case of three or more reaction fields, linking in series and linking in parallel may also be combined.

The temperature and pressure at the reaction field may be set as appropriate for the gas-liquid reaction carried out using the reaction method of the invention.

### <Flow of gaseous starting material and liquid starting material>

The reaction method of the invention includes alternately flowing a gaseous starting material containing a first starting material and a liquid starting material containing a second starting material, to a reaction field holding a solid catalyst. For example, it may include alternately repeating a procedure of gas feed processing in which a gaseous starting material containing the first starting material is supplied to the reaction field for a predetermined time period, and liquid feed processing in which a liquid starting material containing the second starting material is supplied for a predetermined time period.

When the gaseous starting material and liquid starting material are alternately flowed through the reaction field, the first starting material in the gaseous starting material may be adsorbed and held in the solid catalyst which is being held in the reaction field, and then contacted with the second starting material in the flowed liquid starting material, on the solid catalyst. Alternatively, the second starting material in the liquid starting material may be adsorbed and held on the solid catalyst which is being held in the reaction field, and then contacted with the first starting material in the flowed gaseous starting material, on the solid catalyst.

The reaction method of the invention is characterized by alternately flowing a gaseous starting material containing a first starting material and a liquid starting material containing a second starting material, to a reaction field holding a solid catalyst. That the gaseous starting material and liquid starting material are "alternately flowed" means that gas feed processing in which the gaseous starting material is supplied to the reaction field and liquid feed processing in which the liquid starting material is supplied are carried out repeatedly, and that liquid feed processing is not carried out during gas feed processing and gas feed processing is not carried out during liquid feed processing. Alternate flow of the gaseous starting material and liquid starting material in this manner may be carried out during at least part, and preferably all, of the reaction time in which the gas-liquid reaction is carried out.

By such alternating flow, gas feed processing is carried out, and the first starting material in the gaseous starting material is supplied onto the solid catalyst, when liquid feed processing is not being carried out, i.e. when virtually no liquid starting material is present on the solid catalyst (preferably with the solid catalyst merely wetted with the liquid starting material). The first starting material can therefore reach the solid catalyst essentially without requiring its dissolution in the liquid starting material, thereby increasing the reaction efficiency of the first starting material.

Gas feed processing is not carried out, and the first starting material is not supplied onto the solid catalyst, when liquid feed processing is being carried out, i.e. when a significant amount of liquid starting material is present on the solid catalyst and the gaseous first starting material needs to be dissolved in the liquid starting material in order to reach the solid catalyst. This can reduce first starting material consumption that does not contribute to the reaction.

It is conjectured that gas-liquid reaction with a solid catalyst in the reaction method of the invention takes place efficiently by the action mechanism described above, thus increasing the efficiency of use of the starting materials. However, the present invention is not to be constrained by this conjecture.

The flow time and flow volume of the gaseous starting material containing the first starting material and the liquid starting material containing the second starting material, and the supply rates of the first starting material and second starting material, may be appropriately set depending on the type of gas-liquid reaction, the type of reaction field, and the scale of the reaction.

The gaseous starting material containing the first starting material and the liquid starting material containing the second starting material may also be flowed through the reaction field each independently, after preheating or precooling.

The flowing gas volume, the gas temperature, the flow time and the direction of flow for each gas feed processing operation may be the same or different. Likewise, the flowing liquid starting material volume, the liquid starting material temperature, the flow time and the direction of flow for each liquid feed processing operation may also be the same or different. The direction of flow of the gaseous starting material for one gas feed processing operation and the direction of flow of the liquid starting material for its subsequent liquid feed processing may be in the same direction or the opposite direction. Likewise, the direction of flow of the liquid starting material for one liquid feed processing operation and the direction of flow of the gaseous starting material for its subsequent gas feed processing may also be in the same direction or the opposite direction.

The feed rate for the gaseous starting material in each gas feed processing operation in the reaction method of the invention, in terms of the volume of the gaseous starting material at the reaction temperature, is either equal to or greater than the volume of the reaction field. By flowing the gaseous starting material at such a feed rate, the liquid starting material fed during the immediately previous liquid feed processing operation can be purged from the reaction field, thus maximizing the advantageous effect of alternating flow of the gaseous starting material and liquid starting material.

Similarly, the feed rate of the liquid starting material in each liquid feed processing operation, in terms of the volume of the liquid starting material, may be either equal to the volume of the reaction field or greater than the volume of the reaction field. By flowing the liquid starting material at such a feed rate, the gaseous starting material fed during the immediately previous gas feed processing operation can be purged from the reaction field, thus maximizing the advantageous effect of alternating flow of the gaseous starting material and liquid starting material.

In the method of the invention,
each feed rate for the gaseous starting material, in terms of the volume of the gaseous starting material at the reaction temperature, may be set either equal to the volume of the reaction field or greater than the volume of the reaction field, and
each feed rate for the liquid starting material, in terms of the volume of the liquid starting material, may be set either equal to the volume of the reaction field or greater than the volume of the reaction field.

When multiple different first starting materials are used in the gas-liquid reaction, the multiple different first starting materials may be mixed to form a single type of gas mixture for supply to the method of the invention, or they may be prepared separately and each supplied in an alternating manner to the method of the invention. For example, in a gas-liquid reaction using two different first starting materials (first starting material A and first starting material B) and one second starting material, a gaseous starting material containing the first starting material A and the first starting material B and a liquid starting material containing the second starting material may be alternately flowed through the reaction field, or a gaseous starting material containing the first starting material A, a gaseous starting material containing the first starting material B and a liquid starting material containing the second starting material may be flowed in order through the reaction field.

The same procedure may be carried out as described above even when using multiple second starting materials for the gas-liquid reaction, substituting the term "liquid starting material" for "gaseous starting material".

The gaseous starting material containing the first starting material and the liquid starting material containing the second starting material may also be circulated and flowed through the reaction field each independently, after one flow pass through the reaction field.

According to one aspect of the invention, the gaseous starting material containing the first starting material is flowed through the reaction field in one pass.

According to another aspect of the invention, the liquid starting material containing the second starting material is flowed in a circulating manner so that after it has flowed through the reaction field, the flowed liquid starting material is recovered and reflowed.

If either or both the gaseous starting material containing the first starting material and the liquid starting material containing the second starting material are flowed in a circulating manner through the reaction field, the starting material concentrations will be periodically reduced and the product concentration will increase in the gaseous starting material or liquid starting material that has been flowed in a circulating manner. In this case, therefore, the component concentration in the gaseous starting material or liquid starting material may be monitored to determine the progress of the reaction and serve as an indicator of completion of the reaction.

### <Reaction system>

According to another aspect of the invention, a reaction system for carrying out the reaction method of the invention is provided.

The reaction system of the invention is a reaction system having:
a reaction field,
a gaseous starting material housing unit for housing of the gaseous starting material,
a liquid starting material housing unit for housing of the liquid starting material,
a gaseous starting material feeder for flow of the gaseous starting material through the reaction field in an intermittent manner, and
a liquid starting material feeder for flow of the liquid starting material through the reaction field in an intermittent manner.

The reaction field in the reaction system of the invention can be described in the same manner as the reaction field for the reaction method of the invention. The reaction field may comprise a temperature regulator and a pressure regulator.

The gaseous starting material housing unit for housing the gaseous starting material and the liquid starting material housing unit for housing the liquid starting material may each employ an appropriate publicly known vessel such as a tank. The gaseous starting material housing unit and liquid starting material housing unit may each independently be provided with a temperature regulator, pressure regulator, pressure gauge, level meter and sensors.

The gaseous starting material feeder for producing flow of the gaseous starting material through the reaction field in an intermittent manner may be an apparatus comprising an open/close valve and a gas flow regulator. If necessary, it may also comprise a pump and a pressure regulator.

The liquid starting material feeder for producing flow of the liquid starting material through the reaction field in an intermittent manner may be an apparatus comprising an open/close valve, a liquid flow regulator, a liquid feed pump and a pressure regulator.

The reaction system of the invention may have a construction in which the liquid starting material that has been flowed through the reaction field is recovered and reflowed. Such a construction may return the liquid starting material flowed through the reaction field back to the liquid starting material housing unit.

The reaction system of the invention can be suitably used to carry out the reaction method of the invention.

### EXAMPLES

In the following Examples and Comparative Examples, the reaction system illustrated in Fig. 1 was used for reaction. The reaction system comprises a reaction field (1), a gaseous starting material housing unit (2) and a liquid starting material housing unit (4).

The reaction field (1) used was the catalyst apparatus fabricated for each of the Examples and Comparative Examples. A predetermined gaseous starting material (3) is housed in the gaseous starting material housing unit (2), and a predetermined liquid starting material (5) is housed in the liquid starting material housing unit (4).

A gaseous starting material supply valve (V_{G}) is shown as part of the gaseous starting material feeder, while other devices such as the flow regulator are omitted. Likewise, a liquid starting material supply valve (V_{L}) is shown as part of the liquid starting material feeder, while other devices such as the flow regulator and liquid feed pump are omitted.

The reaction system of Fig. 1 is constructed so that the gaseous starting material (3) and liquid starting material (5) are each independently supplied or cut off to the reaction field (1) by opening and closing the gaseous starting material supply valve (V_{G}) and liquid starting material supply valve (V_{L}).

The substrates and reagents used in the Examples and Comparative Examples are as follows.
Honeycomb substrate: cordierite, wall flow type honeycomb substrate, length: 50 mm, diameter: 30 mm, apparent capacity: 35 mL, cell number: 400 cpsi (62 cell/cm²).
Pellet column: glass cylindrical column, length: 50 mm, diameter: 30 mm, apparent capacity: 35 mL.
Alumina 1: γ-alumina, BET specific surface area: 200 m²/g, mean particle diameter (D50): 3.6 µm.
Alumina 2: γ-alumina, BET specific surface area: 170 m²/g, mean particle diameter (D50): 4.2 µm.
Alumina 3: θ-alumina, BET specific surface area: 130 m²/g, mean particle diameter (D50): 4.5 µm.
Barium sulfate 1: BaSOq, BET specific surface area: 20 m²/g, mean particle diameter (D50): 3.7 µm.

### I. Hydrogen reduction of 4-nitrophenol to 4-aminophenol

In Examples 1 to 3 and Comparative Examples 1 to 3, hydrogen reduction of 4-nitrophenol to 4-aminophenol was carried out for comparison between the method of the invention and a method of supplying a conventional gas-liquid mixture.

### <Example 1 and Comparative Example 1>

A honeycomb substrate was coated with an aqueous coating solution containing alumina 1 (1.9 g) and palladium nitrate (0.10 g as metal), and then dried and fired to fabricate a catalyst device containing Pd as the catalyst metal, which was used as reaction field (1).

Hydrogen was housed in the gaseous starting material housing unit (2), as gaseous starting material (3). In the liquid starting material housing unit (4) there was housed 100 mL of a saturated methanol solution of 4-nitrophenol, as liquid starting material (5).

In the reaction system of Fig. 1, the gaseous starting material (3) that has passed through the reaction field (1) is discarded. The liquid starting material (5) that has passed through the reaction field (1), on the other hand, is returned again and reflowed to the liquid starting material housing unit (4), being circulated between the reaction field (1) and the liquid starting material housing unit (4).

### <Example 1>

For Example 1, after the reaction field (1) was adjusted to 35°C, the procedures of:
gas feed processing for 1 minute by opening the gaseous starting material supply valve (V_{G}) and closing the liquid starting material supply valve (V_{L}) to supply the gaseous starting material (3) to the reaction field (1) at a flow rate of 500 mL/min (ntp), and
liquid feed processing for 2 minutes by closing the gaseous starting material supply valve (V_{G}) and opening the liquid starting material supply valve (V_{L}) to supply the liquid starting material (5) to the reaction field (1) at a flow rate of 100 mL/min,
were alternately repeated for a total reaction time of 90 minutes (total gas feed time: 30 minutes, total liquid feed time: 60 minutes).

The feed rate per gas feed processing of the gaseous starting material (3) (hydrogen) during the reaction was 500 mL (ntp) in terms of standard conditions, and the feed rate per liquid feed processing of the liquid starting material (5) was 200 mL, which were both greater than the apparent capacity of the honeycomb substrate (35 mL) used as the reaction field (1).

The total feed rate of the gaseous starting material (3) (hydrogen) during the reaction was 15,000 mL (ntp), and the cumulative feed rate of the liquid starting material (5) was 6,000 mL.

The conversion rate for 4-nitrophenol to 4-aminophenol in the liquid starting material (5) was 87%.

### <Comparative Example 1>

For Comparative Example 1, after adjusting the reaction field (1) to 35°C and opening the gaseous starting material supply valve (V_{G}) and liquid starting material supply valve (V_{L}),
gas feed processing in which the gaseous starting material (3) was supplied at a flow rate of 500 mL/min (ntp), and
liquid feed processing in which the liquid starting material (5) was supplied at a flow rate of 100 mL/min,
were carried out simultaneously for a total of reaction time of 30 minutes.

The total feed rate of the gaseous starting material (3) (hydrogen) during the reaction was 15,000 mL (ntp), and the total feed rate of the liquid starting material (5) was 3,000 mL.

The conversion rate for 4-nitrophenol to 4-aminophenol in the liquid starting material (5) was 69%.

### <Example 2 and Comparative Example 2>

For Example 2 and Comparative Example 2, the reaction field (1) used was a catalyst device produced in the following manner, with reaction conducted in the same manner as Example 1 and Comparative Example 1, respectively.

A honeycomb substrate was coated with an aqueous coating solution containing alumina 2 (1.9 g) and palladium nitrate (0.04 g as metal), and then dried and fired to fabricate a catalyst device containing Pd as the catalyst metal, which was used as reaction field (1).

The conversion rate of 4-nitrophenol to 4-aminophenol in the liquid starting material (5) was 80% in Example 2 and 42% in Comparative Example 2.

### <Example 3 and Comparative Example 3>

For Example 3 and Comparative Example 3, the reaction field (1) used was a catalyst device produced in the following manner, with reaction conducted in the same manner as Example 1 and Comparative Example 1, respectively.

A honeycomb substrate was coated with an aqueous coating solution containing Ba sulfate (2.1 g) and palladium nitrate (0.04 g as metal), and then dried and fired to fabricate a catalyst device containing Pd as the catalyst metal, which was used as reaction field (1).

The conversion rate of 4-nitrophenol to 4-aminophenol in the liquid starting material (5) was 74% in Example 3 and 29% in Comparative Example 3.

### II. Hydrogen reduction of resorcinol to 1,3-cyclohexanedione

In Example 4 and Comparative Example 4, hydrogen reduction of resorcinol to 1,3-cyclohexanedione was carried out for comparison between the reaction method of the invention and a reaction method by supply of a conventional gas-liquid mixture.

### <Example 4 and Comparative Example 4>

The reaction was carried out using the reaction system shown in Fig. 1.

A honeycomb substrate was coated with an aqueous coating solution containing alumina 1 (1.9 g) and palladium nitrate (0.10 g as metal), and then dried and fired to fabricate a catalyst device containing Pd as the catalyst metal, which was used as reaction field (1).

Hydrogen was housed in the gaseous starting material housing unit (2), as gaseous starting material (3). In the liquid starting material housing unit (4) there was housed 100 mL of an aqueous solution of resorcinol at a concentration of 1.8 mol/L, as the liquid starting material (5).

### <Example 4>

For Example 4, after the reaction field (1) was adjusted to 60°C, the procedures of:
gas feed processing for 3 minutes by supplying the gaseous starting material (3) to the reaction field (1) at a flow rate of 10 mL/min (ntp), and
liquid feed processing for 3 minutes by supplying the liquid starting material (5) to the reaction field (1) at a flow rate of 30 mL/min,
were alternately repeated for a total reaction time of 60 minutes (total gas feed time: 30 minutes, total liquid feed time: 30 minutes).

The feed rate per gas feed processing of the gaseous starting material (3) (hydrogen) during the reaction was 30 mL (ntp) in terms of the volume under standard conditions, which corresponded to approximately 37 mL at the reaction temperature (60°C). The feed rate per liquid feed processing of the liquid starting material (5) was 90 mL. The feed rate per gas feed processing of the gaseous starting material and the feed rate per liquid feed processing of the liquid starting material (5) were both greater than the apparent capacity of the honeycomb substrate (35 mL) used as the reaction field (1).

The total feed rate of the gaseous starting material (3) (hydrogen) during the reaction was 300 mL (ntp), and the total feed rate of the liquid starting material (5) was 900 mL.

The conversion rate for resorcinol to 1,3-cyclohexanedione in the liquid starting material (5) during the reaction was 3.8%.

### <Comparative Example 4>

For Comparative Example 4, after adjusting the reaction field (1) to 60°C, the procedures of:
gas feed processing in which the gaseous starting material (3) was supplied to the reaction field (1) at a flow rate of 10 mL/min (ntp), and
liquid feed processing in which the liquid starting material (5) was supplied to the reaction field (1) at a flow rate of 30 mL/min,
were carried out simultaneously for a total of reaction time of 30 minutes.

The total feed rate of the gaseous starting material (3) (hydrogen) during the reaction was 300 mL (ntp), and the total feed rate of the liquid starting material (5) was 900 mL.

The conversion rate for resorcinol to 1,3-cyclohexanedione in the liquid starting material (5) during the reaction was 2.2%.

Table 1 summarizes the results of Examples 1 to 4 and Comparative Examples 1 to 4.

### [Table 1]

**Table 1**

| Reaction type | | | Hydrogen reduction of 4-nitrophenol | | | | | | Hydrogen reduction of resorcinol | |
|---|---|---|---|---|---|---|---|---|---|---|
| Experiment No. | | | Example 1 | Comp. Example 1 | Example 2 | Comp. Example 2 | Example 3 | Comp. Example 3 | Example 4 | Comp. Example 4 |
| Reaction field | Form | | Wall flow honeycomb | Wall flow honeycomb | Wall flow honeycomb | Wall flow honeycomb | Wall flow honeycomb | Wall flow honeycomb | Wall flow honeycomb | Wall flow honeycomb |
| | Catalyst metal | Type | Pd | Pd | Pd | Pd | Pd | Pd | Pd | Pd |
| | | Amount (g) | 0.10 | 0.10 | 0.04 | 0.04 | 0.04 | 0.04 | 0.19 | 0.19 |
| | Support | Type | Alumina 1 | Alumina 1 | Alumina 2 | Alumina 2 | Barium sulfate | Barium sulfate | Alumina 3 | Alumina 3 |
| | | Amount (g) | 1.9 | 1.9 | 1.9 | 1.9 | 2.1 | 2.1 | 3.6 | 3.6 |
| Gas/reaction mixture supply method | | | Alternate switching | Simultaneous continuous | Alternate switching | Simultaneous continuous | Alternate switching | Simultaneous continuous | Alternate switching | Simultaneous continuous |
| Gaseous starting material | First starting material | | Hydrogen | Hydrogen | Hydrogen | Hydrogen | Hydrogen | Hydrogen | Hydrogen | Hydrogen |
| | Flow rate (mL/min (ntp)) | | 500 | 500 | 500 | 500 | 500 | 500 | 10 | 10 |
| | Supply time period (min) | | 3n to 3n+1 | 0 to 30 | 3n to 3n+1 | 0 to 30 | 3n to 3n+1 | 0 to 30 | 6n to 6n+3 | 0 to 30 |
| | Total supply time (min) | | 30 | 30 | 30 | 30 | 30 | 30 | 30 | 30 |
| | Total feed rate (mL) | | 15,000 | 15,000 | 15,000 | 15,000 | 15,000 | 15,000 | 300 | 300 |
| Liquid starting material | Second starting material | | 4-Nitrophenol | 4-Nitrophenol | 4-Nitrophenol | 4-Nitrophenol | 4-Nitrophenol | 4-Nitrophenol | Resorcinol | Resorcinol |
| | Solvent | | Methanol | Methanol | Methanol | Methanol | Methanol | Methanol | Water | Water |
| | Initial reaction mixture volume (mL) | | 100 | 100 | 100 | 100 | 100 | 100 | 100 | 100 |
| | Flow rate (mL/min) | | 100 | 100 | 100 | 100 | 100 | 100 | 30 | 30 |
| | Supply time period (min) | | 3n+1 to 3(n+1) | 0 to 30 | 3n+1 to 3(n+1) | 0 to 30 | 3n+1 to 3(n+1) | 0 to 30 | 6n+3 to 6(n+1) | 0 to 30 |
| | Total supply time (min) | | 60 | 30 | 60 | 30 | 60 | 30 | 30 | 30 |
| | Cumulative feed rate (mL) | | 6000 | 3000 | 6000 | 3000 | 6000 | 3000 | 900 | 900 |
| Total reaction time (min) | | | 90 | 30 | 90 | 30 | 90 | 30 | 60 | 30 |
| Reaction temperature (°C) | | | 35 | 35 | 35 | 35 | 35 | 35 | 60 | 60 |
| Conversion rate (%) | | | 87 | 69 | 80 | 42 | 74 | 29 | 3.8 | 2.2 |

In Table 1, the symbol "n" in "3n to 3n + 1" and "6n to 6n + 3" for the supply time period (minutes) for the gaseous starting material and "3n + 1 to 3(n + 1)" and "6n + 3 to 6(n + 1)" for the supply time period for the liquid starting material, each represents an integer of 0 or greater. That is, when "n" is 0 in Examples 1 to 3, for example, this means that the gaseous starting material was supplied to the reaction field during the period of 0 minutes or longer and 1 minute or less (3 × 0 to 3 × 0 + 1 (min)) from the start of the reaction, and that the liquid starting material was supplied during the period of 1 minute or longer and 3 minutes or less (3 × 0 + 1 to 3 × (0 + 1) (min)).

The results in Table 1 confirmed, by comparison at the point where the total feed rates of the gaseous starting materials were equal, that the conversion rate of the reactant (4-nitrophenol or resorcinol) in the liquid starting material was higher with the reaction method of the invention (Examples 1 to 4), compared to the reaction method by simultaneous gas-liquid supply according to the prior art (Comparative Examples 1 to 4).

The reaction method of the invention was therefore demonstrated to have higher utilization efficiency of the gaseous starting material compared to the reaction method of the prior art.

### III. Comparison of honeycomb substrate and pellet column as reaction field

Examples 5 and 6 were carried out to compare the use of a honeycomb substrate or a pellet column as the reaction field for hydrogen reduction of 4-nitrophenol to 4-aminophenol using the reaction system shown in Fig. 1.

### <Example 5>

Hydrogen reduction of 4-nitrophenol was carried out in the same manner as Example 1, except that a honeycomb substrate was coated with an aqueous coating solution containing alumina 1 (1.615 g) and palladium nitrate (0.085 g as metal), and then dried and fired to fabricate a catalyst device containing Pd as the catalyst metal, which was used as reaction field (1).

For Example 5, the conversion rate for 4-nitrophenol to 4-aminophenol in the liquid starting material (5) was 87%.

### <Example 6>

After mixing alumina 1 (8.075 g) and palladium nitrate (0.085 g as metal) in purified water, the solid portion was collected, dried and fired to obtain a Pd-supported Al catalyst. The Pd-supported Al catalyst was crushed into pellets with a particle size range of 500 µm or greater and 2,000 µm or smaller. Hydrogen reduction of 4-nitrophenol was carried out in the same manner as Example 1, except that the obtained pellets were packed into a pellet column and used as reaction field (1).

For Example 6, the conversion rate for 4-nitrophenol to 4-aminophenol in the liquid starting material (5) was 79%.

Table 2 shows the results for Examples 5 and 6 together with the results for Comparative Example 1.

### [Table 2]

**Table 2**

| Reaction type | | | Hydrogen reduction of 4-nitrophenol | | |
|---|---|---|---|---|---|
| Experiment No. | | | Example 5 | Example 6 | Comp. Example 1 |
| Reaction field | Form | | Wall flow honeycomb | Pellet column | Wall flow honeycomb |
| | Catalyst metal | Type | Pd | Pd | Pd |
| | | Amount (g) | 0.085 | 0.085 | 0.10 |
| | Support | Type | Alumina 1 | Alumina 1 | Alumina 1 |
| | | Amount (g) | 1.615 | 8.075 | 1.9 |
| Gas/reaction mixture supply method | | | Alternate switching | Alternate switching | Simultaneous continuous |
| Gaseous starting material | First starting material | | Hydrogen | Hydrogen | Hydrogen |
| | Flow rate (mL/min (ntp)) | | 500 | 500 | 500 |
| | Supply time period (min) | | 3n to 3n+1 | 3n to 3n+1 | 0 to 30 |
| | Total supply time (min) | | 30 | 30 | 30 |
| | Total feed rate (mL) | | 15,000 | 15,000 | 15,000 |
| Liquid starting material | Second starting material | | 4-Nitrophenol | 4-Nitrophenol | 4-Nitrophenol |
| | Solvent | | Methanol | Methanol | Methanol |
| | Initial reaction mixture volume (mL) | | 100 | 100 | 100 |
| | Flow rate (mL/min) | | 100 | 100 | 100 |
| | Supply time period (min) | | 3n to 3(n+1) | 3n to 3(n+1) | 0 to 30 |
| | Total supply time (min) | | 60 | 60 | 30 |
| | Cumulative feed rate (mL) | | 6000 | 6000 | 3000 |
| Total reaction time (min) | | | 90 | 90 | 30 |
| Reaction temperature (°C) | | | 35 | 35 | 35 |
| Conversion rate (%) | | | 87 | 79 | 69 |

In Table 2, each "n" represents an integer of 0 or greater.

The results in Table 2 confirmed that with the reaction method of the invention, the conversion rate of the reactant (4-nitrophenol) in the liquid starting material was higher, in both Example 5 which used a reaction field comprising a honeycomb substrate and Example 6 which used a reaction field comprising a pellet column, compared to the reaction method by simultaneous gas-liquid supply according to the prior art (Comparative Example 1). The conversion rate of the reactant in the liquid starting material was particularly higher in Example 5 which used a reaction field comprising a honeycomb substrate.

### IV. Oxygen oxidation reaction of cinnamyl alcohol to cinnamaldehyde

In Example 7 and Comparative Example 5, oxygen oxidation reaction of cinnamyl alcohol to cinnamaldehyde was carried out using the reaction system shown in Fig. 1, for comparison between the reaction method of the invention and a reaction method by supply of a conventional gas-liquid mixture.

### <Example 7 and Comparative Example 5>

A honeycomb substrate was coated with an aqueous coating solution containing alumina 1 (1.9 g) and palladium nitrate (0.10 g as metal), and then dried and fired to fabricate a catalyst device containing Pd as the catalyst metal, which was used as reaction field (1).

Oxygen was housed in the gaseous starting material housing unit (2), as gaseous starting material (3). In the liquid starting material housing unit (4) there was housed 100 mL of a saturated toluene solution of cinnamyl alcohol, as liquid starting material (5).

### <Example 7>

For Example 7, after the reaction field (1) was adjusted to 100°C, the procedures of:
gas feed processing for 0.5 minutes by opening the gaseous starting material supply valve (V_{G}) and closing the liquid starting material supply valve (V_{L}) to supply the gaseous starting material (3) to the reaction field (1) at a flow rate of 220 mL/min (ntp), and
liquid feed processing for 4.5 minutes by closing the gaseous starting material supply valve (V_{G}) and opening the liquid starting material supply valve (V_{L}) to supply the liquid starting material (5) to the reaction field (1) at a flow rate of 100 mL/min,
were alternately repeated for a total reaction time of 100 minutes (total gas feed time: 10 minutes, total liquid feed time: 90 minutes).

The feed rate per gas feed processing of the gaseous starting material (3) (oxygen) during the reaction was 110 mL (ntp) in terms of the volume under standard conditions, which corresponded to approximately 150 mL at the reaction temperature (100°C). The feed rate per liquid feed processing of the liquid starting material (5) was 225 mL. The feed rate per gas feed processing of the gaseous starting material and the feed rate per liquid feed processing of the liquid starting material (5) were both greater than the apparent capacity of the honeycomb substrate (35 mL) used as the reaction field (1).

The total feed rate of the gaseous starting material (3) (oxygen) during the reaction was 2,200 mL (ntp), and the cumulative feed rate of the liquid starting material (5) was 4,500 mL.

The conversion rate for cinnamyl alcohol to cinnamaldehyde in the liquid starting material (5) during the reaction was 73%.

### <Comparative Example 5>

For Comparative Example 5, after adjusting the reaction field (1) to 100°C and opening the gaseous starting material supply valve (V_{G}) and liquid starting material supply valve (V_{L}), the procedures of:
gas feed processing in which the gaseous starting material (3) was supplied at a flow rate of 220 mL/min (ntp), and
liquid feed processing in which the liquid starting material (5) was supplied at a flow rate of 50 mL/min,
were carried out simultaneously for a total of reaction time of 10 minutes.

The total feed rate of the gaseous starting material (3) (hydrogen) during the reaction was 2,200 mL (ntp), and the total feed rate of the liquid starting material (5) was 500 mL.

The conversion rate for cinnamyl alcohol to cinnamaldehyde in the liquid starting material (5) during the reaction was 33%.

Table 3 below shows the results for Example 7 and Comparative Example 5.

### [Table 3]

**Table 3**

| Reaction type | | | Oxygen oxidation of cinnamyl alcohol | |
|---|---|---|---|---|
| Experiment No. | | | Example 7 | Comp. Example 5 |
| Reaction field | Form | | Wall flow honeycomb | Wall flow honeycomb |
| | Catalyst metal | Type | Pd | Pd |
| | | Amount (g) | 0.10 | 0.10 |
| | Support | Type | Alumina | Alumina |
| | | Amount (g) | 1.9 | 1.9 |
| Gas/reaction mixture supply method | | | Alternate switching | Simultaneous continuous |
| Gaseous starting material | First starting material | | Oxygen | Oxygen |
| | Flow rate (mL/min (ntp)) | | 220 | 220 |
| | Supply time period (min) | | 5n to 5n+0.5 | 0 to 10 |
| | Total supply time (min) | | 10 | 10 |
| | Total feed rate (mL) | | 2200 | 2200 |
| Liquid starting material | Second starting material | | Cinnamyl alcohol | Cinnamyl alcohol |
| | Solvent | | Toluene | Toluene |
| | Initial reaction mixture volume (mL) | | 100 | 100 |
| | Flow rate (mL/min) | | 50 | 50 |
| | Supply time period (min) | | 5n+0.5 to 5(n+1) | 0 to 10 |
| | Total supply time (min) | | 90 | 10 |
| | Cumulative feed rate (mL) | | 4500 | 500 |
| Total reaction time (min) | | | 100 | 10 |
| Reaction temperature (°C) | | | 100 | 100 |
| Conversion rate (%) | | | 73 | 33 |

In Table 3, "n" represents an integer of 0 or greater.

The results in Table 3 confirmed that the reaction method of the invention is effective for both hydrogen reduction using hydrogen as the gaseous starting material, and oxygen oxidation reaction using oxygen as the gaseous starting material.

### REFERENCE SIGNS LIST

1 Reaction field
2 Gaseous starting material housing unit
3 Gaseous starting material
4 Liquid starting material housing unit
5 Liquid starting material
V_{G} Gaseous starting material supply valve
V_{L} Liquid starting material supply valve

## Claims

1. A reaction method for a gas-liquid reaction using a solid catalyst, wherein the method includes:
alternately flowing a gaseous starting material containing a first starting material and a liquid starting material containing a second starting material through a reaction field holding a solid catalyst, to react the first starting material and second starting material on the solid catalyst.

2. The reaction method according to claim 1, wherein each feed rate for the gaseous starting material is either equal to the volume of the reaction field or greater than the volume of the reaction field, in terms of the volume of the gaseous starting material at the reaction temperature.

3. The reaction method according to claim 1 or 2, wherein each feed rate for the liquid starting material is either equal to the volume of the reaction field or greater than the volume of the reaction field, in terms of the volume of the liquid starting material.

4. The reaction method according to any one of claims 1 to 3, wherein:
the reaction field is a honeycomb substrate, and
the solid catalyst is held on the honeycomb substrate.

5. The reaction method according to any one of claims 1 to 4, wherein the liquid starting material that has been flowed through the reaction field is recovered and reflowed.

6. The reaction method according to any one of claims 1 to 5, wherein:
the first starting material is hydrogen, and
the second starting material is a hydrogen-reducible compound.

7. The reaction method according to any one of claims 1 to 5, wherein:
the first starting material is oxygen or air, and
the second starting material is an oxygen-oxidizable or air-oxidizable compound.

8. A reaction system that carries out the reaction method according to any one of claims 1 to 7, wherein the reaction system has:
a reaction field,
a gaseous starting material housing unit for housing of the gaseous starting material,
a liquid starting material housing unit for housing of the liquid starting material,
a gaseous starting material feeder for flow of the gaseous starting material through the reaction field in an intermittent manner, and
a liquid starting material feeder for flow of the liquid starting material through the reaction field in an intermittent manner.

9. The reaction system according to claim 8, which has a construction in which the liquid starting material that has been flowed through the reaction field is recovered and reflowed.
